# EUROPEAN PATENT APPLICATION

(11) **EP 4 317 417 A1**
(43) Date of publication of application: **07.02.2024**
(21) Application number: 22827353.8
(22) Date of filing: 02.06.2022
(51) Int. Cl.: C12N 5/073, C12N 5/071, C12N 5/02

(54) **WAYNE293 LVPRO CELLS ADAPTED TO SERUM-FREE MEDIUM ENVIRONMENT AND USE THEREOF**

(30) Priority: 22.06.2021 CN 202110688920
(71) Applicant: Quacell Biotechnology, Co. Ltd., Zhongshan, Guangdong 528437 (CN)
(72) Inventor: YU, Xiaoyu, Zhongshan, Guangdong 528437 (CN); PAN, Honghui, Zhongshan, Guangdong 528437 (CN); YUAN, Jun, Zhongshan, Guangdong 528437 (CN); XU, Wenyan, Zhongshan, Guangdong 528437 (CN); XIE, Guohao, Zhongshan, Guangdong 528437 (CN); WU, Fan, Zhongshan, Guangdong 528437 (CN)
(74) Representative: Murgitroyd & Company
(86) International application number: PCT/CN2022/096765
(87) International publication number: WO 2022/267856

(57) **Abstract**

The present invention discloses a WAYNE293 LVPRO cell adapted to a serum-free medium environment, and applications thereof. The human embryonic kidney cell WAYNE 293 is preserved in China General Microbiological Culture Collection Center (CGMCC) on May 24, 2021, the address is Institute of Microbiology, Chinese Academy of Sciences, No. 3, No. 1 Yard, Beichen West Road, Chaoyang District, Beijing, the postal code is 100101, and the preservation number is CGMCC No. 22348. The human embryonic kidney cell WAYNE 293 provided by the present invention may be derived from suspension culture of HEK293 cells in a serum-free medium free of an anti-agglomeration agent, has the characteristics of short doubling time, high growth density, high virus packaging efficiency, and is an important support for industrial development in the field of cell and gene therapy.

## Description

### TECHNICAL FIELD

The present invention belongs to the field of cells, and more particularly relates to a WAYNE293 LVPRO cell adapted to a serum-free medium environment, with short doubling time, high cell growth density and high virus packaging efficiency, and applications thereof.

### BACKGROUND

With the development of cell and gene therapy industry, a method of introducing a target gene using a virus vector has become a new direction for people to treat congenital genetic diseases, leukemia, cancers and other major diseases.

At present, the virus vector is mainly prepared from HEK293 as a host cell by means of a method of using a cationic polymer or other chemical reagents to perform transient transfection. This is because HEK293 has certain advantages in transient transfection efficiency and virus packaging yield compared to other cells. However, adherent culture is still used for most of the genes and cell therapy drugs that have been on the market currently. For example, a method of fetal bovine serum + basal medium is adopted in the process of HKE293 cell culture. The adherent culture has insurmountable difficulties in the later stage of industrialization. The number of cells is subject to a surface area of a culture flask, so the equipment investment and labor input will greatly increase the preparation cost of the virus vector if the production scale of the virus vector is expected to increase in the process of large-scale industrial production. Meanwhile, animal-derived fetal bovine serum still needs to be added in the adherent culture process at present as an essential cell culture raw material, which will bring inevitable risks in the safety of genes and cell therapy products.

In addition, on an existing technical platform for serum-free suspension culture of HEK293, the dosage of an anti-agglomeration agent in the medium will generally be reduced or even completely canceled for the pursuit of subsequent transient transfection efficiency. However, the HEK293 cell as a human embryonic kidney cell is very easy to agglomerate in the process of serum-free suspension culture, which greatly affects the cell viability, density and transfection efficiency.

Therefore, finding an HEK293 cell that may be subjected to suspension culture in a serum-free medium free of an anti-agglomeration agent and has short doubling time, high growth density, high virus packaging efficiency and other characteristics is an important support for the industrial development in the field of cell and gene therapy.

### SUMMARY

A first object of the present invention is to provide an HEK293 cell, i.e., an HEK293 cell-human embryonic kidney cell WAYNE 293, that may be subjected to suspension culture in a serum-free medium free of an anti-agglomeration agent and has short doubling time, high growth density, high virus packaging efficiency.

The human embryonic kidney cell WAYNE 293 of the present invention is preserved in China General Microbiological Culture Collection Center (CGMCC) on May 24, 2021, the address is Institute of Microbiology, Chinese Academy of Sciences, No. 3, No. 1 Yard, Beichen West Road, Chaoyang District, Beijing, the postal code is 100101, and the preservation number is CGMCC No. 22348.

A second object of the present invention is to provide an application of the human embryonic kidney cell WAYNE 293 as a host cell in the protein expression and growth, the expression and production of a virus vector or the vaccine production.

The protein can be an antibody.

A third object of the present invention is to provide an application of the human embryonic kidney cell WAYNE 293 as a host cell in the preparation of a drug in the field of cell or gene therapy.

The human embryonic kidney cell WAYNE 293 provided by the present invention may be derived from suspension culture of an HEK293 cell in a serum-free medium free of an anti-agglomeration agent, has the characteristics of short doubling time, high growth density, high virus packaging efficiency, and is an important support for industrial development in the field of cell and gene therapy.

The human embryonic kidney cell WAYNE 293 is preserved in China General Microbiological Culture Collection Center (CGMCC) on May 24, 2021, the address is Institute of Microbiology, Chinese Academy of Sciences, No. 3, No. 1 Yard, Beichen West Road, Chaoyang District, Beijing, the postal code is 100101, and the preservation number is CGMCC No. 22348.

### BRIEF DESCRIPTION OF THE DRAWINGS

FIG. 1 shows a screening process of a WAYNE293 LVPRO cell of the present invention;
FIG. 2 is a diagram showing a monoclonal growth condition in a 96-well plate;
FIG. 3 is a growth curve of a WAYNE 293LVPRO cell and an HEK293 cell;
FIG. 4 shows an LV vector expression yield of a WAYNE293 monoclonal cell screened by a high-throughput system;
FIG. 5 shows an LV virus expression yield of a WAYNE293 LVPRO cell, wherein RUN1, RUN2, and RLTN3 respectively represent expression results repeated three times under the same conditions, proving that the results have good reproducibility, high stability and credibility; and
FIG. 6 shows a comparison of the WAYNE293 LVPRO cell and an original HEK293 cell in terms of virus packaging efficiency.

### DETAILED DESCRIPTION OF THE EMBODIMENTS

The following embodiments are further illustrations of the present invention, rather than limitations to the present invention.

### Embodiment 1:

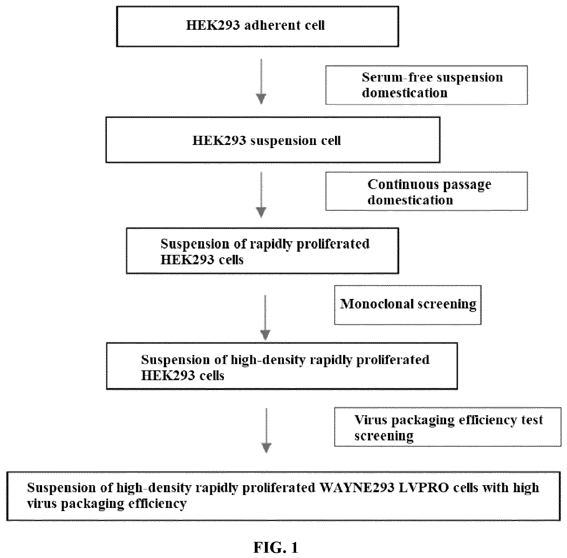

A screening process of a WAYNE 293 LVPRO cell of the present invention is shown in FIG. 1. The specific steps are as follows.
1. An adherent HEK293 cell (ECACC 85120602) is subjected to serum-free suspension domestication in a medium (QuaCelQUACELL CD02 medium, purchased from QUACELL BIOTECHNOLOGY, CO. LTD.), so that the HEK293 cell is adapted to a suspension growth condition.

The specific domestication methods are as follows.

### A. Cell resuscitation

Key equipment: shaker, biological safety cabinet

A cell resuscitation method is as follows: a medium is placed in a water bath at 36.5 to 37.5°C for 30 to 40 min. A stably-passaged HEK293 cell population cryotube is quickly placed in the water bath at 36.5 to 37.5 °C, and slowly shaken to completely melt. 75% ethanol is sprayed onto the surface of the cryotube, and the cyrotube is then transferred into a biological safety cabinet that has been irradiated with ultraviolet rays (≥30 min). A cell fluid is pipetted and transferred to a 50 ml centrifuge tube, added with 5 ml of medium and mixed well, and centrifuged for 5 min at 1000 rpm. A supernatant is discarded. Then, a total of 20 ml of DMEM medium (QUACELL) + 10% (final concentration volume fraction) fetal bovine serum (QUACELL) that have been added in the water bath is pipetted into the centrifuge tube, and the cell fluid is resuspended. The cell suspension is then transferred to a T75 shake flask, shaken evenly and sampled, transferred to a CO₂ incubator and stands for culture under the conditions shown in Table 1. A daily sampling interval is controlled within 24 hours ± 2 hours. Cell passage is performed at the passaging density of 5E5 cells/mL when the cell density reaches 1-2E6 cells/mL and the viability rate is greater than 90%.

**Table 1**

| Cell resuscitation | |
|---|---|
| Process parameters | Set value (acceptable standard) |
| Culture temperature | 37°C (36.5 to 37.5°C) |
| CO₂ concentration | 5% (4 to 6%) |
| Water bath temperature in cryotube | 37°C (36.5 to 37.5°C) |

### B. Suspension domestication (culture in T75 culture flask)

Suspension domestication (cultured in T75 culture flask ) method is as follows: the medium is heated in a water bath at 36.5 to 37.5°C for 30 to 40 min. The resuscitated HEK293 cells obtained in step A are taken. 20 ml of cell fluid is pipetted from the T75 culture flask and transferred to a 50 ml centrifuge tube, added with 5 ml of domestication medium and mixed well, and centrifuged for 5 min at 1000 rpm. A supernatant is discarded. Then, 20 ml of domestication medium that has been added in the water bath is pipetted into a centrifuge tube, and the cell fluid is resuspended. The cell suspension is then transferred to a T75 shake-flask, shaken well and sampled, transferred to a CO₂ incubator and stands for culture under the conditions shown in Table 2. A daily sampling interval is controlled within 24 hours ± 2 hours. Cell passage is performed at the passaging density of 5E5 cells/mL when the cell density reaches 1-2E6 cells/mL and the viability rate is greater than 90%. The domestication medium is CD02 medium (QUACELL) + 10% (final concentration volume fraction) fetal bovine serum (QUACELL), which are mixed well.

During the passage process, the amount of serum used in the medium is gradually reduced, and the reduced-serum domestication is performed at a level of 10% (10% of the serum content) reduction in each passage until the serum content in the medium is 0. The next passage is transferred to a QUACELL CD02 medium for suspension culture. The cell state is observed, the viability rate is maintained above 90% after multiple passages, and then the domestication is completed. Therefore, HEK293 cells adapted to suspension growth are obtained.

**Table 2**

| Suspension domestication (T75 flask culture) | |
|---|---|
| Process parameters | Set value (acceptable standard) |
| Culture temperature | 37°C (36.5 to 37.5°C) |
| CO₂ concentration | 5% (4 to 6%) |
| CD02 medium | |

2. The continuous passage of HEK293 cells under suspension growth shortens the doubling time of HEK293 from the initial 48 hours to about 26 hours, while the maximum cell density may be up to 1 ×10⁷ cells/mL.

The specific steps are as follows.

### A. 125 ml shake-flask culture

A 125 ml shake-flask culture method is as follows: 20 ml of HEK293 cell fluid with a density of 1*E6 cells/mL under suspension growth is transferred to a 125 ml cell culture shake flask, added with a WAYNE293 medium such that the cell fluid expands to 30 ml, then shaken evenly and sampled, and cultured in a shaker under the conditions as shown in Table 3; the cells are sampled and counted every day; the cell density and viability data are recorded; and cell passage is performed at the passage density of 5E5 cells/mL when the cell density reaches 1-2E6 cells/mL and the viability rate is greater than 90%.

**Table 3**

| 125 ml shake-flask culture | |
|---|---|
| Process parameters | Set value (acceptable standard) |
| Culture temperature | 37°C (36.5 to 37.5°C) |
| CO₂ concentration | 5% (4 to 6%) |
| Shaker speed | 125 rpm (110 to 140 rpm) |

### B. 500 ml shake-flask culture

Key equipment: shaker, biological safety cabinet

A 500 ml shake-flask culture method is as follows: 30 mL of cell fluid in a 125 ml shake-flask is transferred to a 500 ml cell culture shake flask, added with a WAYNE293 medium such that the cell fluid expands to 120 ml, then shaken evenly and sampled, and cultured in a shaker under the conditions shown in Table 4. The cells are sampled and counted every day; the cell density and viability data are recorded; and cell passage is performed at the passage density of 5E5 cells/mL when the cell density reaches 1-2E6 cells/mL.

**Table 4**

| 500 ml shake-flask culture | |
|---|---|
| Process parameters | Set value (acceptable standard) |
| Culture temperature | 37°C (36.5 to 37.5°C) |
| CO₂ concentration | 5% (4 to 6%) |
| Shaker speed | 125 rpm (110 to 140 rpm) |

### C. Shake-flask culture

Key equipment: shaker, biological safety cabinet

The culture method is as follows: 120 mL of cell fluid in the 500 ml shake-flask is transferred to a 1 L cell culture shake-flask, added with a WAYNE293 medium such that the cell fluid expands to 400 ml, then shaken evenly and sampled, and cultured in a shaker under the conditions shown in Table 5; the cells are sample and counted every day; the cell density and viability data are recorded; and cell passage is performed at the passage density of 5E5 cells/mL when the cell density reaches 1-2E6 cells/mL.

**Table 5**

| 1 L shake-flask culture | |
|---|---|
| Process parameters | Set value (acceptable standard) |
| Culture temperature | 37°C (36.5 to 37.5°C) |
| CO₂ concentration | 5% (4 to 6%) |
| Shaker speed | 125 rpm (110 to 140 rpm) |

As a result, the doubling time of the suspended HEK293 cells is shortened from the initial 48 hours to about 26 hours, while the maximum cell density may be up to 1 ×10⁷ cells/mL.

### D. Aliquoting cryopreservation

Key equipment: biological safety cabinet, liquid nitrogen tank, desk-type horizontal centrifuge, 2-8°C refrigerator, -20°C refrigerator, and -80°C refrigerator

An aliquoting cryopreservation method is as follows: at the end of cell culture, trypsin-EDTA is added for digestion so that the cells are no longer attached to the surface of the culture flask; the cell fluid is collected, and centrifuged for 5 min at 1000 rpm; a supernatant is discarded, and a centrifuged precipitate is collected; and the centrifuged precipitate is resuspended with a cryopreservation solution, such that the required resuspended cell density reaches 1.3×10⁷ cells/mL to 1.8×10⁷ cells/mL (cryopreservation solution formula: 90% fresh WAYNE293 medium + 10% DMSO). The resuspension is aliquoted in cryopreservation tubes, 1 ml each, labeled and placed in a cooling box to cool down in an accepted cooling order. The cooling box is placed in an -80°C refrigerator and kept for about 24 hours. After being cooled in the -80°C refrigerator, the cooling box is stored in a liquid nitrogen tank which is managed in a double-person double-lock mode.

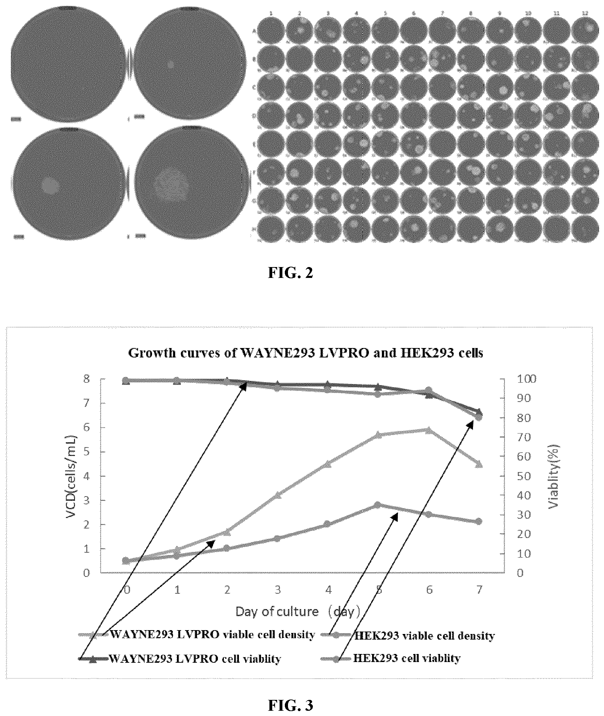

3. A limited dilution is performed for the HEK293 cells obtained in step 2 by using a QUACELL's CD02 medium, and the cells are diluted to a density of 5 cells/mL. Then, a 96-well plate is subjected to monoclonal seeding by using a single-cell printing technology, and the monoclonality of the cells is determined by a cell imaging system, such that more than 3000 monoclonal cells of HEK293 suspension cells may be obtained. Some of these monoclonal cells exhibit rapid growth characteristics in the 96-well plate. These fast-growing HEK293 cells are collected (FIG. 2).

4. The fast-growing HEK293 cells collected in step 3 are tested for virus packaging efficiency by a high-throughput screening system; after the test, the cells with high packaging efficiency are selected for passage culture; and the HEK293 cells after passage culture are tested for virus packaging efficiency by using the high-throughput screening system, and this step is repeated for several times.

The fast-growing HEK293 cells collected in step 3 are suspended by using a 96-well deep well plate and tested for virus packaging efficiency; and the cells are subjected to passage culture by using a QUACELL's WAYNE293 medium, and then tested for virus packaging efficiency.

The specific method is as follows:

### A. Virus packaging efficiency testing

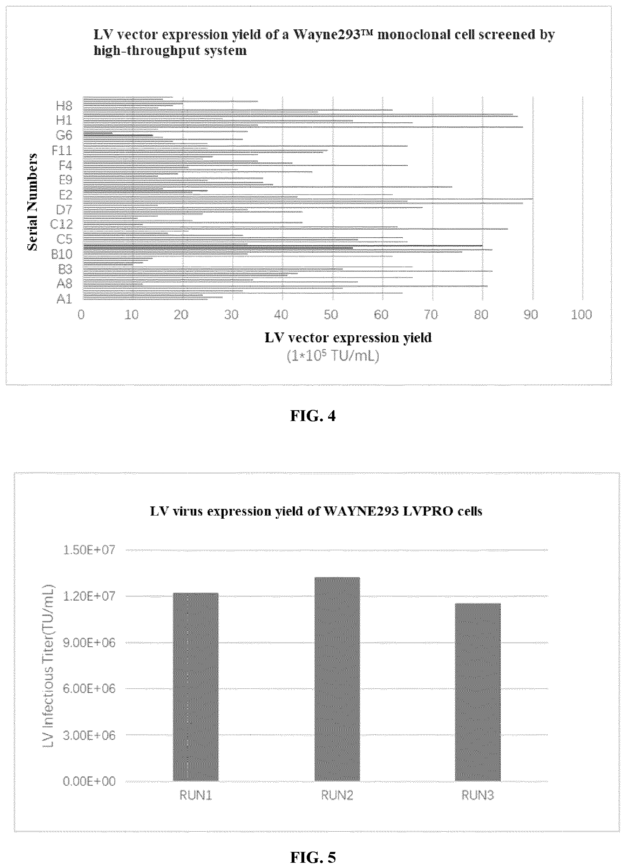

1 ml of HEK293 cells with a density of 1-2E6 cells/mL in the 96-well deep well plate is transiently transfected at a ratio of 1ug of plasmid: 4ul of PEI, by using a tetra-plasmid virus packaging system (Invitrogen ViraPower^{™} Lentiviral Expression Systems) with a GFP fluorescent protein label, and a supernatant is collected after 48 hours; the collected supernatant is then added to the adherent 293T cells prepared in advance according to a volume ratio of 1: 10; the 293T cells are infected with a lentivirus in the supernatant, such that a target gene (GFP) in the lentivirus is introduced into the 293T cells; and the titer level of the virus expressed in the 96-well deep well plate is determined (calculation formula: TU/ml = (F×Ci/V)×D) according to a proportion of a GFP green fluorescent protein expressed in the 293T cells, thereby determining the virus packaging efficiency of the monoclonal cells in the 96-well plate (FIG. 4).

### B. The passage culture is as follows:

Passage culture in a 125 ml shake-flask

Key equipment: CO₂ incubator, biological safety cabinet

A 125 ml shake-flask culture method is as follows: the cell fluid of the monoclonal cells with high virus packaging efficiency in the 96-well plate is transferred to a 125 ml cell culture shake-flask, added with a Wayne293[A1] medium such that the cell fluid expands to 30 ml, then shaken evenly and sampled, and cultured in a shaker under the conditions shown in Table 6; and the cell passage is performed at the passage density of 5E5 cells/mL when the cell density reaches 1-2E6 cells/mL.

**Table 6**

| 125 ml shake-flask culture | |
|---|---|
| Process parameters | Set value (acceptable standard) |
| Culture temperature | 37°C (36.5 to 37.5°C) |
| CO₂ concentration | 5% (4 to 6%) |
| Shaker speed | 125 rpm (110 to 140 rpm) |

Then, the HEK293 suspension cells with short doubling time, high cell growth density and high virus packaging efficiency are screened out in combination with the cell doubling time and the maximum density of cell growth, and named human embryonic kidney cells WAYNE 293, hereinafter abbreviated: WAYNE 293 LVPRO cells.

5. Comparison of performances of WAYNE 293 LVPRO cells of the present invention and original HEK293 cells

A. The WAYNE 293 LVPRO cells of the present invention and original HEK293 cells are cultured in batches in a WAYNE293 medium, wherein their cell growth curves are shown in FIG. 3, and their doubling time is 22 to 24 hours.

Batch culture method:

Key equipment: CO₂ incubator, biological safety cabinet

The batch culture method is as follows: 1 mL of resuscitated cell fluid with a density of 1E7 cells/mL is transferred to a 125 ml cell culture shake flask, added with a Wayne293 medium such that the cell fluid expands to 30 ml, shaken evenly and sampled, and cultured in a shaker according to the conditions shown in Table 7, and samples are taken daily for statistics.

**Table 7**

| Batch culture | |
|---|---|
| Process parameters | Set value (acceptable standard) |
| Culture temperature | 37°C (36.5 to 37.5°C) |
| CO₂ concentration | 5% (4 to 6%) |
| Shaker speed | 125 rpm (110 to 140 rpm) |

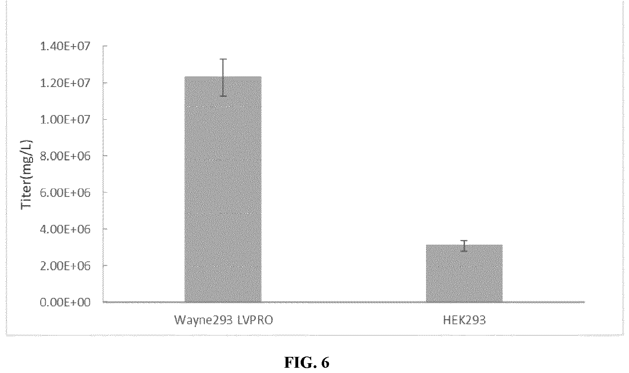

B. The WAYNE 293 LVPRO cells and the original HEK293 cells are tested for virus packaging efficiency by using the high-throughput screening system in step 4, and the results are shown in FIGS. 5 and 6. As can be seen from FIGS. 5 and 6, the LV virus packaging efficiency of the WAYNE 293 LVPRO cells of the present invention is much greater than that of the original HEK293 cells.

The human embryonic kidney cell WAYNE 293 is preserved in China General Microbiological Culture Collection Center (CGMCC) on May 24, 2021, the address is Institute of Microbiology, Chinese Academy of Sciences, No.3, No.1 Yard, Beichen West Road, Chaoyang District, Beijing, the postal code is 100101, and the preservation number is CGMCC No.22348.

### Embodiment 2:

The human embryonic kidney cells WAYNE 293 are used for vaccine production according to the following specific steps:

### 1. Treatment of human embryonic kidney cells WAYNE 293

### 1.1 Cell resuscitation

(1) The cells are shaken rapidly (< 2 min) in 37°C water to thaw a cell fluid in a cryotube;
(2) The cell fluid is transferred to a 15 ml centrifuge tube, added with 5 ml of preheated AAV^{™} Tranfection Medium, and centrifuged for 5 minutes at 1000 rpm; a supernatant is discarded; the cells are resuspended with 5 ml of AAV^{™} Tranfection Medium for cell counting;
(3) all cell fluid in the centrifuge tube is transferred to a 125 ml shake-flask filled with 25 ml of preheated AAV^{™} Tranfection Medium, or diluted to the desired cell density;
(4) the cells are cultured in a humidified CO₂ shake incubator containing 5% CO₂ at 37°C, and a cap is unscrewed (or a vent cap is used) during culture for gas exchange; and
(5) The cells are cultured for 2 to 3 days after cell resuscitation, and passaged when the cells are in the middle of logarithmic growth; The resuscitated cells should be passaged at least three times before other experiments.

### 1.2 Passage culture of cells

(1) The cell passage is performed when the cell density reaches 1.5×10⁶ to 2.5×10⁶ cells/mL, and the cells are counted using an automatic cell counter or other counting instruments, followed passage according to a desired inoculum density; and
(2) the recommended inoculum density in a seed bottle is 3 ×10⁵ to 5×10⁵ cells/mL; the cells are continuously cultured in the CO₂ shake incubator containing 5% CO₂ at 37°C, and the next passage can be performed usually 2 to 3 days later.

### 1.3 Cell cryopreservation

(1) The WayneLVProTM HEK293 suspension cells are frozen at a final density of 1×10⁷ to 1.5×10⁷ cells/ml, and 90% AAV^{™} Tranfection Medium and 10% DMSO are mixed to a total volume of 1 ml;
(2) the cell density is allowed to be 1.5×10⁶ to 2.5×10⁶ cells/mL before cryopreservation and harvesting of the cells, and the survival rate of cells after digestion is terminated is greater than 95%;
(3) the cells are centrifuged for 5 min at 1000 rpm until precipitate is generated, a culture supernatant is discarded and replaced with a pre-cooled AAV^{™} Tranfection Medium containing 10% DMSO, and the cells are gently blown and beaten with a pipette to resuspend the cells;
(4) the cells are adjusted to a final concentration of 1.5×10⁷ cells/mL and aliquoted into cryotubes at a dosage of 1 ml each;
(5) the cells are frozen in an automatic or manually controlled programmed cooling device according to standard procedures; when the ideal procedure indicates cooling, the freezing rate should be reduced by 1°C per minute; and
(6) the frozen cryotubes are transferred to liquid nitrogen for long-term storage.

### 2. Preparation of virus

### 2.1 Cell preparation

A bottle of human embryonic kidney cells WAYNE293 in good condition after about 2 to 3 passages is passaged according to a density of 0.5E + 06 cells/mL. On the third day, a counter is used to record the cell density. If the cell density has reached 2.5 to 3.0E + 06 cells/mL, the cells may expand according to the above passage rules. The cells expand to a reactor working volume according to the actual production line design, while paying attention to maintaining the cell viability and maintaining the cell density in the logarithmic growth phase.

### 2.2 Virus inoculation

A seed virus for vaccine preparation, which is usually a lyophilized product, shall be qualified in terms of virulence, minimum immunization, safety and sterility, and shall be verified and distributed by the vaccine virus seed preservation department designated in China.

The taken seed virus should be used as a virus seed after cell subculture adaptation according to regulations. The virus seeds for vaccine production, which are wet virus seeds or freeze-dried virus seeds, should be controlled within a certain number of passages according to regulations.

Virus inoculation culture is divided into synchronous culture and asynchronous culture, wherein the former cells are inoculated with virus seeds while or shortly after the cells are aliquoted, to culture, e.g., cat or dog infectious enteritis vaccines (parvovirus); and the latter cells are inoculated with virus seeds after the cells form a monolayer, to culture, e.g., an epidemic encephalitis B cell culture vaccine. After the cells form the monolayer, a culture solution is usually poured away. The cells are inoculated with virus seeds at a dosage of 1% to 2%, adsorbed and then added with a maintenance solution for continuous culture, till the cells are harvested when more than 70% to 85% of cells have cytopathic effects.

The harvesting time and method of the virus culture solution depend on the nature of a vaccine, the virus solution may be harvested 5 to 7 times during the virus proliferation culture process, and the cell virus solution is supplied for vaccine preparation after being qualified under sterility test and virus titer determination. The virus can proliferate in large quantities in the human embryonic kidney cells 293.

### 2.3 Virus harvesting and purification

Depending on different viruses, after the harvested supernatant or cell precipitate is qualified under sterility test and virus titer determination, an equilibrium solution and an inactivator (formaldehyde, phenol, crystal violet, etc.) are added in prescribed proportions to prepare a homogenate, which is then inactivated according to an inactivation temperatures and time of different viruses. For example, a rabies vaccine is prepared by adding glycerol and 1% phenol distilled water to brain tissues at a ratio of 1:4 and inactivating at 36°C for 7 days; and a swine fever crystal violet vaccine is prepared by mixing 4 parts of blood poison and 1 part of crystal violet glycerol solution, and inactivating at 37°C to 38°C for 6 to 8 days.

### 2.4 Verification of vaccines

According to the characteristics of the vaccines themselves, the safety and efficacy of the vaccines are verified, and their experimental methods and quality standards may refer to the prior art.

## Claims

1. A human embryonic kidney cell WAYNE 293, having a preservation number of CGMCC No. 22348.

2. An application of the human embryonic kidney cell WAYNE 293 according to claim 1 as a host cell in protein expression and growth.

3. The application according to claim 2, wherein the protein is an antibody.

4. The application of the human embryonic kidney cell WAYNE 293 according to claim 1 as a host cell in the expression and production of a virus vector.

5. The application of the human embryonic kidney cell WAYNE 293 according to claim 1 in vaccine production.

6. The application of the human embryonic kidney cell WAYNE 293 according to claim 1 as a host cell in the preparation of a drug in the field of cell or gene therapy.
